# EUROPEAN PATENT SPECIFICATION

(11) **EP 2 512 267 B1**
(45) Date of publication and mention of the grant of the patent: **07.10.2015**
(21) Application number: 10795173.3
(22) Date of filing: 13.12.2010
(51) Int. Cl.: A23L 1/308, A23F 5/26, A61K 31/702, A61P 3/04, A23L 2/52, A23L 1/30

(54) **COFFEE TREATMENT METHOD**
KAFFEEBEHANDLUNGSVERFAHREN
PROCÉDÉ DE TRAITEMENT DU CAFÉ

(30) Priority: 14.12.2009 GB 0921826
(43) Date of publication of application: 24.10.2012
(73) Proprietor: Kraft Foods R & D, Inc., Deerfield, IL 60015 (US)
(72) Inventor: KENNY, Francisco Javier Silanes, Banbury Oxfordshire OX16 3LQ (GB); FERNANDES, Jose Arthur, Banbury Oxfordshire OX16 3LQ (GB); LECLERC, Vincent, Montreal Québec H4N 2Y1 (CA)
(74) Representative: Boult Wade Tennant
(86) International application number: PCT/US2010/060053
(87) International publication number: WO 2011/075431

(56) References cited:
- WO-A2-2008/011562
- JP-A- 5 168 411
- US-A1- 2008 213 425
- US-A1- 2009 005 342

## Description

The present invention relates to a method for producing a concentrated mannose oligosaccharide from a coffee material, in particular from a coffee extraction residue material. More particularly, the invention involves a process for treating a hydrolyzed coffee extraction residue material to obtain mannose-oligosaccharides.

### Background

Mannose-oligosaccharides are known for use as food additives. Furthermore, it is known that mannose-oligosaccharides having a degree of polymerisation from 5 to 10 have health benefits such as reducing body fat and especially abdominal fat. Mannose-oligosaccharides may be produced by the hydrolysis of mannan materials since this breaks the mannan chains of up to DP40 into the useful shorter chains. Coffee is known to be a material rich in mannans.

There are many different techniques for hydrolysing mannans. For example, U.S. Pat. No. 2,573,406 discloses a process for producing a soluble coffee which involves hydrolyzing a portion of coffee grounds in a suspension of about 1% sulphuric acid at 100°C for about 1 hour, adjusting the pH of the hydrolysate, filtering the hydrolysate, and drying the extract. In another, similar process described in U.S. Pat. No. 2,687,355, phosphoric acid is used in place of sulphuric acid. Still in another process, disclosed in the U.S. Pat. No. 3,224,879 either alkaline or acid hydrolysis is carried out directly in the extraction train of coffee grounds that have been at least atmospherically extracted.

Thermal hydrolysis is also a well known technique. However, when coffee is thermally hydrolysed under conditions suitable to produce a MOS-rich product, undesirable impurities are formed which are associated with the product. Specifically, extensive hydrolysis in a percolator will produce tars and other impurities. These lead to sour and unpleasant notes, or off-flavours, in the taste of the product. Accordingly, while the MOS-rich product produced by thermal hydrolysis can be introduced into strong flavoured products, such as coffee beverages, it is not suitable for less strongly flavoured products as an additive.

It is known in the art to use activated carbon, adsorptive resins, ion-exchange resin, ion-exchange membranes, and combinations thereof to remove some impurities from the MOS product, but these are time consuming and expensive to perform. Furthermore, they do not fully remove the undesirable off-notes produced by thermal hydrolysis.

Accordingly, there is a desire for a process for providing a MOS-rich additive for use in foodstuffs and beverages which addresses at least some of the disadvantages associated with the prior art or provides a useful alternative process for doing so.

US 2008/213425 A1 relates to mannooligosaccharide compositions and foods and beverages containing mannooligosaccaharides. JP 5 168411 A relates the extraction of a coffee component from extraction residue of coffee. WO 2008/011562 A2 relates to a composition for treating diabetes comprising oligosaccharides comprising mannose as a constituent sugar. US 2009/005342 A1 relates to a composition having a blood pressure reducing and/or elevation suppressing effect in mammals, which comprises, as the main component, oligosaccharides composed of constituent monosaccharides mainly comprising mannose in an oral ingestible form.

According to a first aspect, the present disclosure provides a process for obtaining a precipitate from coffee, the precipitate having greater than 50 dry weight percent mannose-oligosaccharides having a degree of polymerisation (DP) of from 1 to 10 (MOS), the method comprising the steps of:
(i) providing a MOS-containing hydrolysate derived from a coffee extraction residue material;
(ii) contacting the MOS-containing hydrolysate with an organic solvent to form a suspension; and
(iii) recovering a precipitate,
wherein the organic solvent is added in an amount of at least 50% v/v to the hydrolysate in liquid form, and wherein the organic solvent is methanol, ethanol, n-propanol, isopropanol, or a combination of two or more thereof.

A product comprising the precipitate obtained by the process of the present disclosure is preferably a foodstuff or a beverage. The process described herein can be used to reduce and preferably remove off-flavours from a coffee extraction residue material, the coffee extraction material being for use as an additive in a beverage or foodstuff. More particularly, the process described herein can be used to provide a flavourless precipitate for use as an additive in a beverage or foodstuff. The precipitate obtained by the process of the present disclosure can be used for the manufacture of a medicament for reducing body fat levels.

The present disclosure will now be further described. In the following passages different aspects of the disclosure are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

"Mannan" as used herein refers broadly to any polysaccharide consisting of d- mannose units. The monosaccharide d-mannose is an aldohexose and isomer of d-glucose, differing only by having the opposite steric arrangement of the hydroxyl group nearest the carbonyl. Mannan found in the coffee extraction residue material may have up to 40 d-mannose units in a polysaccharide chain, and typically is linked by beta 1-4 glycosidic linkage, identical to those found in cellulose polymer. Because coffee mannan is essentially a linear polymer with similar bonding forces to cellulose, it is also a difficult polymer to hydrolyze. However, under specific reaction conditions, the mannan fraction can be hydrolyzed without affecting the remaining cellulose fraction.

"Degree of Polymerization" or "DP" refers to the number of mono-saccharide units that make up a given mannose-oligosaccharide. Thus, a mannose- oligosaccharide of DP 4 for example consists of 4 mannose units.

"Mannose-oligosaccharide", or the abbreviation MOS, is intended to include mannose and manno-saccharides up to manno-decaose (DP10). A mannose- oligosaccharide having a DP of 1 is technically a monosaccharide but is referred to as an oligosaccharide as mixtures of oligosaccharides may include some monosaccharide units. The mannose-oligosaccharides will often include a plurality of oligosaccharides with different degrees of polymerization.

MOS may be produced by the hydrolysis of mannan. Sources of mannan include raw coffee beans, roasted coffee beans, spent coffee grounds. Any types of coffee beans from any source may be utilized. Examples of coffee that can be used include Arabica, Robusta, Liveria and the like. A single type of coffee or blends of different types of coffee may be utilized. Coffee beans of inferior quality, or size, including those having little or no commercial value, may also be used.

"Coffee extraction residue material" which is be employed to produce the MOS-containing material is intended to mean a roast and ground coffee material that has been at least partially extracted. A coffee extraction residue material is usually obtained from a commercial coffee percolation system. Coffee that has been partly thermally hydrolyzed in order to hydrolyze the less stable polysaccharides such as arabinogalactan is particularly useful as coffee extraction residue material. The spent grounds from a commercial percolation system is an example of coffee that has been atmospherically extracted and partly thermally hydrolyzed, such that about 35 to 60% of the starting roasted coffee grounds has been extracted, typically about 50%. For the sake of simplicity, the "Coffee extraction residue material" is also referred to as "coffee material" herein.

A "hydrolysate" is the product of a hydrolysing step. In this way, mannan-containing coffee materials are processed to produce MOS from the mannan fraction. That is, a hydrolysis process is used to hydrolyze mannan in a coffee material which may have a DP of 10 to 40 or higher to form oligosaccharides having a DP of 1 to 10. Hydrolysis of the mannan material may be conducted using hydrolysis methods that may include acid hydrolysis, thermal hydrolysis, enzyme hydrolysis, microbial fermentation hydrolysis, and mixtures thereof. Thermal hydrolysis is especially preferred due to the simplicity of the processing steps required and the speed of processing. Furthermore, unlike acid hydrolysis, no further steps such as neutralisation are required.

Enzyme hydrolysis may be conducted by suspending mannan material in an aqueous medium and adding appropriate commercially available enzymes, such as for example, cellulase and hemicellulase. Enzyme hydrolysis may be carried out using standard conditions as known by one of ordinary skill in the art.

Microbial fermentation may also be used to hydrolyze mannan material. Mannan material may be fermented with microorganisms that produce enzymes capable of hydrolyzing mannan. Microorganisms which produce enzymes such as cellulase and hemicellulase may be used. One example of an appropriate microorganism is Basidiomycota.

The present disclosure produces a precipitate rich in mannose-oligosaccharides (MOS) from coffee. By rich in MOS it is meant that the precipitate is predominantly MOS. That is, greater than 50%, more preferably greater than 75% and most preferably greater than 90%. Ideally the precipitate is substantially all MOS. That is, greater than 95%, more preferably greater than 98% and most preferably all MOS except unavoidable impurities. All percentages are by weight of the dry component in the mixture.

The process of the present disclosure comprises at least three steps. These are:
(i) providing a MOS-containing hydrolysate derived from a coffee extraction residue material;
(ii) contacting the MOS-containing hydrolysate with an organic solvent to form a suspension; and
(iii) recovering a precipitate.

It will be understood that while these steps are necessarily to be carried out in sequential order, they may form part of a continuous process in which any of the steps may be occurring simultaneously or overlapping to some extent. The process may be performed continuously or batchwise.

The present inventors have discovered that surprisingly it is possible to remove the off-flavours from a hydrolysate, in particular a thermal hydrolysate, by using a solvent extraction technique. Furthermore, this has the dual benefit of providing an improved flavour as well as resulting in a concentration of the more desirable DP5 to DP10 MOS.

The term "organic solvent" as used herein includes any carbon-containing solvent. Preferably the solvent is substantially polar, that is, it has a dielectric constant at 25°C of at least 15. Preferably the solvent is approved and safe for human consumption. The solvent is methanol, ethanol, n-propanol, isopropanol, or a combination of two or more thereof. Ethanol is the most preferred solvent.

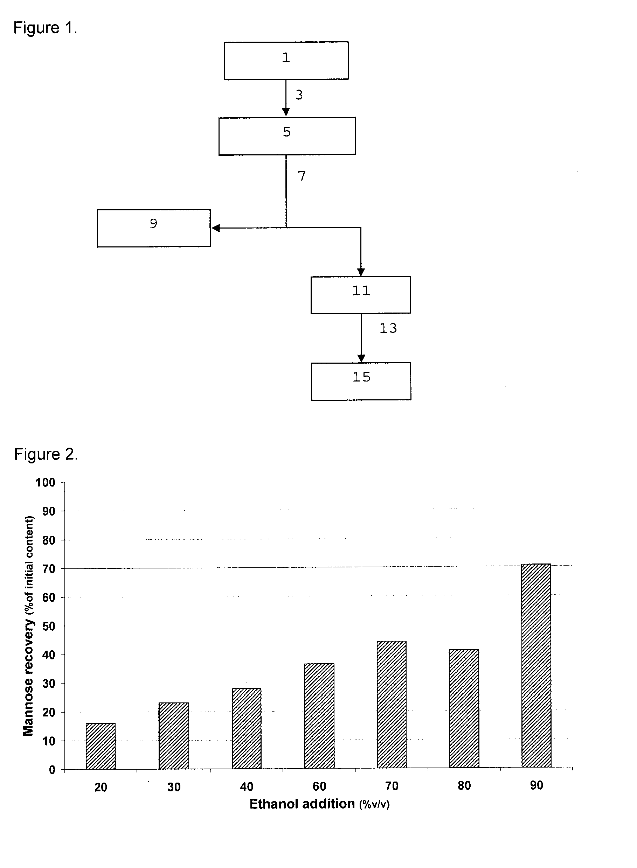

The solvent is preferably added as an excess to ensure that the majority of the MOS is recovered. The proportion added can be measured in terms of the %v/v of the solvent added to the hydrolysate in liquid form. The solvent is added as at least 50% v/v, more preferably at least 70% and most preferably at least 90%. Figure 2 shows examples of how the recovery rate varies according to the proportion of ethanol added. In order to ensure high processing yields it is preferred that no more than 99% v/v solvent is used.

The MOS-containing hydrolysate derived from a coffee material is preferably at least partially dissolved in water before being contacted by the organic solvent. Preferably the hydrolysate is fully dissolved so as to form a mixture and eventually a precipitate when mixed with the organic solvent. This prevents undesirable solid impurities becoming mixed with the precipitate, although these can be pre-sedimented out or removed by any conventional technique.

The step (iii) of recovering the precipitate may be conducted using any conventional technique for separating solids form a liquid. For example, by evaporation, decanting or filtration, and optionally a centrifuging step, and any combination of two or more of the foregoing. Preferably the technique involves a reduction in the temperature of the suspension since this increases the speed at which the precipitate forms and increases the fraction of MOS that precipitates out. The reduction in the temperature may be as a result of natural cooling, especially if the MOS-containing hydrolysate is still warm from a thermal hydrolysis step. Alternatively, the mixture may be initially warmed to allow for cooling. The cooling may be active in order to increase the processing speed and reduce the time taken to produce the product. Active cooling techniques are well known in the art.

The inventors have discovered that the present process advantageously increases the proportion of DP5-DP10 MOS to DP1-DP10 MOS in the precipitate relative to the proportion of DP5-DP10 MOS to DP1-DP10 MOS in the MOS- containing hydrolysate. Preferably the increase is at least 50%, more preferably at least 100% and most preferably at least 200%. Without wishing to be bound by theory, this may be because the shorter chain mannose-oligosaccharides which are of greater solubility in water than the longer chain oligosaccharides, remain in any water fraction present in the suspension. Since they remain dissolved in this way, they do not form part of the retained precipitate.

The precipitate obtained in the method of the present disclosure may still be damp or moist after recovery from the suspension. Accordingly, a further drying step may be included. Drying steps are well known in the art and any suitable drying step may be performed. Especially suitable processes are carried out under a reduced pressure. Drying processes include, but are not limited to, spray drying and freeze-drying.

A further purification step may be used involving with activated carbon, adsorptive resins, ion-exchange resin, ion-exchange membranes, and combinations thereof. Desalting and de-acidification may be conducted using ion-exchange resins and/or ion-exchange membranes. Combinations of these methods may also be used. Further purification may be conducted at higher dosage levels or when the hydrolysate is to be used in certain type of foods or beverages.

The soluble solids obtained by as a precipitate according to the present process may be used as a food or beverage additive, for example, in coffee. Furthermore, in view of the reported health benefits of DP5-DP10 MOS the precipitate may be included in such products as a health-boosting additive. In particular, the precipitate obtainable by the process of the present disclosure may be used for the manufacture of a medicament for reducing body fat levels. The medicament may take the form of a foodstuff or beverage. Suitable food stuffs include dairy products, baked goods and ready meals, as well as pre-prepared beverages or in compositions for preparing instant beverages. Preferably the food or beverage is not a coffee flavoured foodstuff or beverage.

It is preferred that the MOS-containing hydrolysate is prepared in a process of hydrolyzing a coffee material, such as partially extracted roasted and ground coffee, in a thermal hydrolysis step. This may be carried out in a reactor by a high temperature short time process, preferably without the introduction of any added acid catalyst. If acid catalyst is added this complicates the process and the product may need further neutralisation. A tubular plug flow reactor is convenient, although any reactor providing for the relatively high temperature, short time reaction will suffice. The time/temperature relationship is chosen to cause solubilisation and then hydrolysis of the native mannan oligomers from a range of about DP 10 to DP 40 to a range of about DP 1 to DP 10.

The coffee material is subjected to a temperature from about 150°C to 300°C, preferably 200 °C to about 260 ° C, for a period of time ranging from about 1 minute to about 15 minutes in a reactor, to hydrolyze the mannan fraction to about the DP 1 to DP 10 range. After hydrolysis, the soluble MOS-containing hydrolysate can be removed from any solid impurities remaining from the coffee material.

Preferably the coffee material is at least partially hydrated in a liquid, typically water before being fed to a reactor, preferably a plug flow reactor. This makes handling the material easier since it forms a slurry. The hydrated coffee material should be uniform, that is, it should be distributed evenly throughout. If it is made up in batch beforehand, steps should be taken to insure uniformity, such as recirculation by means of a slurry pump. If the hydrolysis reaction is to occur within a plug flow reactor, it is preferable to utilize a slurry which should be between 5 and 25% by weight, most preferably between 10% and 20% by weight, of the dry basis coffee material. When utilizing the plug flow reactor, if the concentration of the slurry exceeds 25% by weight, the slurry becomes too thick to insure proper flow. In the event a different reactor, such as an extruder, is used, it is generally not necessary to prepare a slurry. For example, spent grounds from a conventional percolation system, typically containing between about 65% and 80% by weight liquid, may be fed directly to such an extruder without further dilution. Spent ground containing from about 40% to 65% by weight liquid may also be used. Such grounds can have been partially dehydrated such as by screw pressing, air drying or other methods known in the art.

Suitable continuous reactors include those capable of promoting relatively high temperature, short time reactions, such as single or double screw extruders or plug flow tubular reactors. A suitable batch reactor is a so-called pressure containment vessel, for example an autoclave, or an explosion puffer wherein the coffee extraction residue material is placed in the reactor vessel which is then pressurized and heated, as with steam. The pressure is suddenly and explosively released, discharging the contents from the reactor vessel. The soluble solids are then leached with water from the material so discharged from said reactor vessel. The plug flow tubular reactors are especially convenient. A plug flow tubular reactor is essentially a cylindrical length of pipe in which a reaction can take place. An orifice or other suitable device is placed on the discharge end of the reactor in order to control the pressure in the reactor as well as the rate of discharge from said reactor. "Plug flow" refers to the velocity profile of the slurry flowing through the reactor. Normally, a fluid exhibits a parabolic profile velocity wherein the fluid in the centre of the conduit has a higher velocity than fluid flowing closer to the wall. In an ideal plug flow reactor, the velocity profile is flat, arising from the geometry of the vessel and the nature of the fluid thus assuring the same high temperature, short time reaction conditions for all material in the reactor by minimizing variations in residence time.

The elevated temperature is achieved in the reactor in any of several ways. For example, the slurry may be passed through a heat exchanger as a part of, or separate from the reactor chamber. Temperature may then be maintained by simply insulating the reactor. Alternatively, high pressure steam may be injected directly into the reactor as a means of raising the temperature. Although the steam may dilute the slurry somewhat, such heating is extremely rapid, permitting short reaction times. Selection of the preferred heating method, as well as sizing of the diameter of the reactor and orifice are all within the skill of a worker in the art, based on standard design principles.

The time/temperature conditions maintained within the reactor are, of course, critical in insuring that mannan hydrolysis will occur. It is known that excessive temperatures and pressures increase undesirable tars and off-flavours. However, the treatment process of the present disclosure allows these to be more readily removed. This means higher temperatures and pressures than are conventionally employed may be used.

It has been found that the reaction temperature should be between about 150°C and 300°C, preferably from about 200 ° C to about 260 ° C and most preferably from about 210 ° C to 240 ° C, in order to solubilise and hydrolyze the mannan fraction to the desired range. At least 50% of the mannan fraction is removed from the coffee residue, preferably 75% and more preferably 90%. Such temperatures are preferably combined with a pressure in the reactor of between atmospheric pressure to 100 atmospheres and more preferably between about 20 atmospheres and about 40 atmospheres. This helps increase the yield.

The desired reaction time has been found to be between about 1 minutes and about 15 minutes, preferably from about 2 to about 8 minutes, to effect said hydrolysis.

At any given temperature within the temperature range of the present inventive process, the mannan will be solubilised and hydrolyzed. Yields will increase with residence time up to a maximum and thereafter the yield will reduce as the oligomers degrade to produce either volatiles or insolubles i.e. tars or sludges. The kinetics of the present reaction will generally double with each 10 ° C rise in temperature. At the upper temperature range, the residence times must fall within the lower end of the specified time range; vice-versa, at the lower temperature range, the residence time must fall within the upper end of the time range.

The slurry is preferably passed out of the furnace rapidly to reduce the pressure to which the slurry is subjected to about atmospheric. Such a rapid reduction of pressure causes expansion and evaporative cooling of the slurry thereby "quenching" or immediately terminating the hydrolysis and browning reactions. By so quenching the reaction, it is possible to control the hydrolysis reaction time to within the prescribed 1 to 15 minute period with great reliability.

Separation may be by any method of solid-liquid separation known in the art. For example, said slurry may be filtered in order to remove the hydrolyzed partially extracted roasted and ground coffee therefrom. Alternatively, the slurry may be separated by centrifuging the slurry, as in a basket centrifuge. The MOS-containing hydrolysate used in the present disclosure is preferably formed from the soluble elements of the slurry since these contain the bulk of the mannose-oligosaccharides.

The disclosure is further illustrated in the examples below and with reference to the following figures:
Figure 1 is a flow chart showing steps which may be performed in the process of the present disclosure.
Figure 2 shows how the percentage by weight of mannose recovered (by % of initial content) from a hydrolysate derived from a coffee material varies according to the amount of ethanol added (%v/v).
Figure 3 shows the relative DP fractions present in an unprocessed hydrolysate in g/100g (light grey) compared to a precipitate formed by a process according to the present disclosure in g/100g at 80% v/v ethanol (black).

### Examples

An amount of coffee extraction residue material was mixed with water to form a slurry (10% by weight solids) and passed into a plug flow reactor. The slurry was heated to 230°C for 6 minutes. After this thermal hydrolysis step, the solids were extracted (by centrifuging) from the slurry and discarded to leave a liquid hydrolysate 1 (see the flow chart in Figure 1).

The liquid hydrolysate 1 was then mixed in a mixing step 3 with sufficient ethanol (food-grade) to form an 80% v/v ethanolic solution 5. The mixing step 3 was performed on a continuous liquid hydrolysate 1 stream passing from the plug flow reactor.

The ethanolic solution 5 was allowed to cool to encourage the MOS fraction to precipitate out. After precipitation the suspension was centrifuged in a separation step 7. The separation step 7 produced a supernatant 9 which was discarded and a precipitate 11.

The precipitate 11 was then re-dissolved in minimal water and freeze-dried in a finishing step 13. This produced a de-flavoured MOS enriched extract 15. The extract 15 was found to be substantially tasteless.

The extract 15 and the hydrolysate 1 were analysed using the following analytical methods:
▪ Carbohydrate analysis was conducted using an anion exchange system after acid hydrolysis with sulphuric acid and amperometric detection.
▪ Degree of polymerization (DP) analysis was performed using capillary electrophoresis system coupled with a Diode-Array Detector (DAD).

The mannose recovery results for precipitation of hydrolysate at different levels of ethanol addition are shown in Figure 2. It can be seen that as the ethanol content increases the recovery increases.

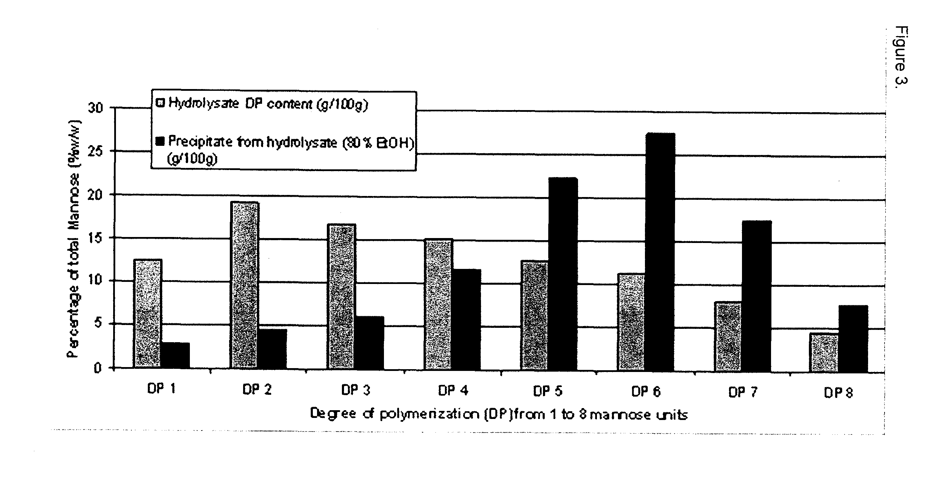

In Figure 3 the DP distribution for both hydrolysate 1 and precipitate extract 15 are shown. For the precipitate extract 15 the majority of mannose recovered was between DP 5-8 (see the Table 1 below). The degree of concentration of DP 5 to 8 in the precipitate extract 15 was about 200% of the hydrolysate 1. The precipitate extract 15 was free of initial off-flavours which were removed during the process.

**Table 1**

| | |
|---|---|
| Sum DP5-DP8 Hydrolysate | 36.35 |
| Sum DP5-DP8 Hydrolysate precipitate | 74.96 |
| Percentage increase (hydrolysate/precipitate) | 206% |

| | |
|---|---|
| - DP5-DP8 values expressed as a percentage. | |

The precipitate recovered in the foregoing example was then introduced into a coffee beverage product and subjected to consumer testing. The objective of the testing was to confirm that the ethanol precipitation could significantly reduce the acidity and bitterness perception in coffee.

2 cups of coffee were presented simultaneously to assessors in white paper cups, under red light (to limit appearance bias).The tasters tasted each cup in individual booths and circled the one being the most acidic and then the most bitter (samples can be re-tasted). 35 semi-trained participated.

Compared to a conventional cup of coffee, 31 tasters selected the cup of coffee containing the precipitate of the present disclosure as being less acidic and 28 found it less bitter. Thus, the sample obtained through ethanol precipitation can be considered as significantly less sour and less bitter at 95% confidence.

The foregoing detailed description has been provided by way of explanation and illustration.

## Claims

1. A process for obtaining a precipitate from coffee, the precipitate having greater than 50 dry weight percent mannose-oligosaccharides having a degree of polymerisation (DP) of from 1 to 10 (MOS), the method comprising the steps of:
(i) providing a MOS-containing hydrolysate derived from a coffee extraction residue material;
(ii) contacting the MOS-containing hydrolysate with an organic solvent to form a suspension; and
(iii) recovering a precipitate,
wherein the organic solvent is added in an amount of at least 50% v/v to the hydrolysate in liquid form, and wherein the organic solvent is methanol, ethanol, n-propanol, isopropanol, or a combination of two or more thereof.

2. A process according to claim 1, wherein the MOS-containing hydrolysate derived from a coffee material is at least partially dissolved in water before step (ii).

3. A process according to any of the preceding claims, wherein step (iii) involves a reduction in the temperature of the suspension and separation of the precipitate from the suspension.

4. A process according to claim 3, wherein separation of the precipitate from the suspension is performed by evaporation, decanting or filtration, and optionally includes an initial centrifuging step.

5. A process according to any of the preceding claims, wherein the proportion MOS having a degree of polymerisation (DP) of 5-10 to the proportion MOS having a DP of 1-10 in the precipitate is at least 50% greater than the proportion of DP5-DP10 MOS to DP1-DP10 MOS in the MOS-containing hydrolysate.

6. A process according to any of the preceding claims, wherein the MOS-containing hydrolysate is prepared by the steps of:
(a) providing a coffee extraction residue material; and
(b) thermally hydrolysing the coffee material to form a MOS-containing hydrolysate.

7. A process according to claim 6, wherein the step of thermally hydrolysing the coffee material is carried out under one or more of the following conditions:
(1) at a temperature of from 150°C to 300°C;
(2) without the addition of an acid catalyst; and / or
(3) under a pressure of from atmospheric pressure to 100 atmospheres.

8. A process according to claim 7, wherein the reaction is carried out at
(i) a temperature of from 200°C to 260°C; and/or
(ii) a pressure of from 20 to 40 atmospheres.

9. A process according to any of the preceding claims, wherein the process further comprises the step of:
(iv) drying the precipitate.

10. A process according to claim 9, wherein the drying step is carried out under a reduced pressure.

11. A process according to any of the preceding claims, wherein the process further comprises the step of including the precipitate as an additive in a beverage or foodstuff.

## Patentansprüche

1. Verfahren zum Erhalten eines Niederschlags aus Kaffee, wobei der Niederschlag mehr als 50 Trockengewichtsprozent Mannose-Oligosaccharide mit einem Polymerisationsgrad (DP) von 1 bis 10 (MOS) aufweist, wobei das Verfahren die Schritte umfasst von:
(i) Bereitstellen eines MOS-enthaltenden Hydrolysats, das von einem Kaffeeextraktions-Rückstandsmaterial abstammt;
(ii) in Kontakt bringen des MOS-enthaltenden Hydrolysats mit einem organischen Lösungsmittel um eine Suspension zu bilden; und
(iii) Wiedergewinnen eines Niederschlags,
wobei das organische Lösungsmittel in einer Menge von mindestens 50% v/v zu dem Hydrolysat in flüssiger Form zugegeben wird, und wobei das organische Lösungsmittel Methanol, Ethanol, n-Propanol, Isopropanol, oder eine Kombination von zwei oder mehreren davon ist.

2. Verfahren nach Anspruch 1, wobei das MOS-enthaltende Hydrolysat, das von einem Kaffeematerial abstammt, vor Schritt (ii) mindestens teilweise in Wasser gelöst wird.

3. Verfahren nach einem der vorhergehenden Ansprüche, wobei Schritt (iii) eine Verringerung der Temperatur der Suspension und Trennung des Niederschlags von der Suspension umfasst.

4. Verfahren nach Anspruch 3, wobei Trennung des Niederschlags von der Suspension durch Evoporieren, Dekantieren oder Filtrieren durchgeführt wird, und optional einen initialen Zentrifugationsschutt umfasst.

5. Verfahren nach einem der vorhergehenden Ansprüche, wobei der Anteil MOS mit einem Polymerisationsgrad (DP) von 5-10 zu dem Anteil MOS mit einem DP von 1-10 im Niederschlag mindestens 50% größer ist als der Anteil von DP5-DPlO MOS zu DP1-DP10 MOS in dem MOS-enthaltenden Hydrolysat.

6. Verfahren nach einem der vorhergehenden Ansprüche, wobei das MOS-enthaltende Hydrolysat hergestellt wird durch die Schritte von:
(a) Bereitstellen eines Kaffeeextraktions-Rückstandsmaterials; und
(b) thermisches Hydrolysieren des Kaffeematerials um ein MOS-enthaltendes Hydrolysat zu bilden.

7. Verfahren nach Anspruch 6, wobei der Schritt des thermischen Hydrolysierens des Kaffeematerials unter einer oder mehreren der folgenden Bedingungen durchgeführt wird:
(1) bei einer Temperatur von 150°C bis 300°C;
(2) ohne den Zusatz eines Säurekatalysators; und/oder
(3) unter einem Druck von atmosphärischem Druck bis 100 Atmosphären.

8. Verfahren nach Anspruch 7, wobei die Reaktion durchgeführt wird bei
(i) einer Temperatur von 200°C bis 260°C; und/oder
(ii) einem Druck von 20 bis 40 Atmosphären.

9. Verfahren nach einem der vorhergehenden Ansprüche, wobei das Verfahren weiter den Schritt umfasst von: (iv) Trocknen des Niederschlags.

10. Verfahren nach Anspruch 9, wobei der Trocknungsschritt bei einem verminderten Druck durchgeführt wird.

11. Verfahren nach einem der vorhergehenden Ansprüche, wobei das Verfahren weiter den Schritt des Zufügens des Niederschlags als ein Zusatz in ein Getränk oder Nahrungsmittel umfasst.

## Revendications

1. Procédé d'obtention d'un précipité à partir de café, le précipité ayant plus de 50 pour cent en poids sec d'oligosaccharides-mannose ayant un degré de polymérisation (DP) allant de 1 à 10 (MOS), le procédé comprenant les étapes qui consistent :
(i) à fournir un hydrolysat contenant des MOS dérivé d'une matière de résidu d'extraction de café ;
(ii) à mettre en contact l'hydrolysat contenant des MOS avec un solvant organique pour former une suspension ; et
(iii) à récupérer un précipité,
dans lequel le solvant organique est ajouté en une quantité d'au moins 50 % v/v à l'hydrolysat sous forme liquide, et où le solvant organique est le méthanol, l'éthanol, le n-propanol, l'isopropanol, ou une combinaison de deux solvants ou plus de ceux-ci.

2. Procédé selon la revendication 1, dans lequel l'hydrolysat contenant des MOS dérivé d'une matière de café est au moins partiellement dissous dans l'eau avant l'étape (ii).

3. Procédé selon l'une des revendications précédentes, dans lequel l'étape (iii) implique une réduction de la température de la suspension et la séparation du précipité de la suspension.

4. Procédé selon la revendication 3, dans lequel la séparation du précipité de la suspension est réalisée par évaporation, décantation ou filtration, et comporte facultativement une étape de centrifugation initiale.

5. Procédé selon l'une des revendications précédentes, dans lequel la proportion MOS ayant un degré de polymérisation (DP) allant de 5 à 10 par rapport à la proportion MOS ayant un DP allant de 1 à 10 dans le précipité est supérieure d'au moins 50 % à la proportion de MOS de DP5 à DP10 par rapport aux MOS de DP1 à DP10 dans l'hydrolysat contenant des MOS.

6. Procédé selon l'une des revendications précédentes, dans lequel l'hydrolysat contenant des MOS est préparé par les étapes qui consistent :
(a) à fournir une matière de résidu d'extraction de café ; et
(b) à hydrolyser thermiquement la matière de café pour former un hydrolysat contenant des MOS.

7. Procédé selon la revendication 6, dans lequel l'étape qui consiste à hydrolyser thermiquement la matière de café est effectuée dans une ou plusieurs des conditions suivantes :
(1) à une température allant de 150°C à 300°C ;
(2) sans l'ajout d'un catalyseur acide ; et/ou
(3) sous une pression allant de la pression atmosphérique à 100 atmosphères.

8. Procédé selon la revendication 7, dans lequel la réaction est effectuée à
(i) une température allant de 200°C à 260°C ; et/ou
(ii) une pression entre 20 et 40 atmosphères.

9. Procédé selon l'une des revendications précédentes, dans lequel le procédé comprend en outre l'étape qui consiste :
(iv) à sécher le précipité.

10. Procédé selon la revendication 9, dans lequel l'étape de séchage est effectuée sous une pression réduite.

11. Procédé selon l'une des revendications précédentes, dans lequel le procédé comprend en outre l'étape qui consiste à inclure le précipité en tant qu'additif dans une boisson ou un produit alimentaire.
